# FASCICULE DE BREVET EUROPEEN

(11) **EP 1 861 389 B1**
(45) Date de publication et mention de la délivrance du brevet: **25.06.2008**
(21) Numéro de dépôt: 06726092.7
(22) Date de dépôt: 15.03.2006
(51) Int. Cl.: C07D 401/06, A61K 31/472, A61P 19/02

(54) **SEL BESYLATE DE LA 7-(2-(4-(3-TRIFLUOROMETHYL-PHENYL)-1,2,3,6-TETRAHYDRO-PYRID-1-YL)ETHYL) ISOQUINOLEINE, SA PREPARATION ET SON UTILISATION EN THERAPEUTIQUE**
7-(2-(4-(3-TRIFLUORMETHYLPHENYL)-1,2,3,6-TETRAHYDROPYRID-1-YL)ETHYL)ISOCHINOLIN-BESYLATSALZ, DESSEN HERSTELLUNG UND DESSEN THERAPEUTISCHE VERWENDUNG
7-(2-(4-(3-TRIFLUOROMETHYL-PHENYL)-1,2,3,6-TETRAHYDRO-PYRID-1-YL)ETHYL) ISOQUINOLINE BESYLATE SALT, PREPARATION AND THERAPEUTIC USE THEREOF

(30) Priorité: 17.03.2005 FR 0502611
(43) Date de publication de la demande: 05.12.2007
(73) Titulaire: Sanofi-Aventis, 75013 Paris (FR)
(72) Inventeur: BARRE, Corinne, F-34560 Montbazin (FR); MONNIER, Olivier, F-34560 Villeveyrac (FR)
(74) Mandataire: Kugel, Dominique
(86) Numéro de dépôt international: PCT/FR2006/000566
(87) Numéro de publication internationale: WO 2006/097624

(56) Documents cités:
- WO-A-01/29026
- WO-A-97/01536
- WO-A-02/085887
- WO-A-02/085888

## Description

L'invention se rapporte à un nouveau sel de la 7-(2-(4-(3-trifluoromethyl-phenyl)-1,2,3,6-tetrahydro-pyrid-1-yl)ethyl) isoquinoleine, à sa préparation et à son utilisation en thérapeutique.

La 7-(2-(4-(3-trifluoromethyl-phenyl)-1,2,3,6-tetrahydro-pyrid-1-yl)ethyl) isoquinoleine, de formule (I) ci-après, est décrite dans le document WO 2001/029026. 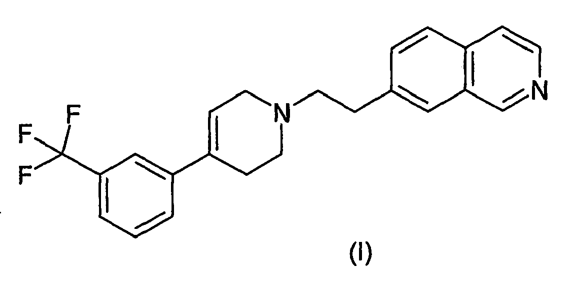 Ce composé, parmi d'autres dérivés de tetrahydro-pyridines, est décrit comme une molécule modulatrice de l'activité du TNF-alpha (de l'anglais Tumor Necrosis Factor).

Le document WO 2001/029026 décrit la 7-(2-(4-(3-trifluoromethyl-phenyl)-1,2,3,6-tetrahydro-pyrid-1-yl)ethyl) isoquinoleine sous forme de base libre ou de différents sels, tels que le dichlorhydrate, le fumarate.

On a maintenant trouvé que la forme salifiée mono benzène sulfonate (encore appelée bésylate) de ce même composé présente des propriétés avantageuses qui la rende particulièrement adaptée à son utilisation en tant que principe actif dans un médicament.

L'invention a donc pour objet le sel bésylate de 7-(2-(4-(3-trifluoromethyl-phenyl)-1,2,3,6-tetrahydro-pyrid-1-yl)ethyl) isoquinoleine, sa préparation et son application en thérapeutique.
Le sel bésylate de 7-(2-(4-(3-trifluoromethyl-phenyl)-1,2,3,6-tetrahydro-pyrid-1-yl)ethyl) isoquinoleine est défini dans la formule (II) ci-après.

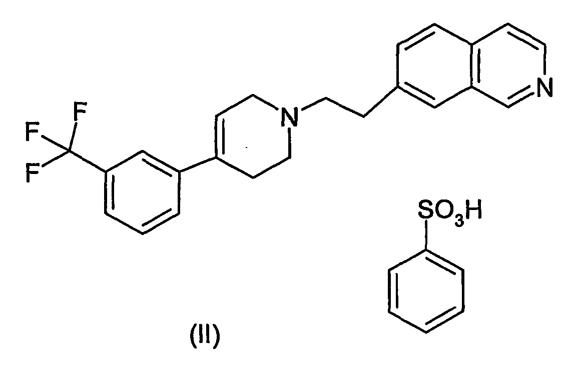

De façon inattendue, on a en effet mis en évidence que la forme bésylate du composé de formule (I) possédait des propriétés de stabilité, notamment par rapport à l'humidité, encore améliorées par rapport à la forme dichlorhydrate ou fumarate de ce même composé.

On a mis également en évidence que la forme bésylate du composé de formule (I) possédait de plus des propriétés d'hygroscopicité améliorées par rapport aux formes dihydrogénosulfate, dibésylate ou dichlorhydrate de ce même composé.

Conformément à l'invention, on peut préparer le composé de formule générale (I) selon le procédé décrit dans la demande WO 2001/029026. Dans ce qui suit, les matières de départ et les réactifs, quand leur préparation n'est pas décrite, sont disponibles dans le commerce ou décrits dans la littérature, ou bien peuvent être préparés selon des méthodes qui y sont décrites ou qui sont connues de l'Homme du métier.

Les avantages liés à la forme bésylate du composé de formule (I) par rapport à la forme base libre ou à d'autres formes salines, tel que le dichlorhydrate et le fumarate, ressortiront des analyses physico-chimiques décrites ci-après.

### Exemple 1 : Préparation du bésylate de 7-(2-(4-(3-trifluoromethyl-phenyl)-1,2,3,6-tetrahydro-pyrid-1-yl)ethyl) isoquinoleine

3,86 g d'acide benzène sulfonique sont dissous sous agitation à 70°C dans 50 mL d'éthanol. 11 g de 7-(2-(4-(3-trifluoromethyl-phenyl)-1,2,3,6-tetrahydro-pyrid-1-yl)ethyl) isoquinoleine dans 60 mL d'éthanol sont ajoutés sous agitation à la solution éthalonique d'acide benzène sulfonique. La solution est ensuite refroidie de 70°C à 20°C, avec un gradient thermique de -20°C par heure.
Le précipité recueilli est ensuite filtré, lavé deux fois avec 22 mL d'éthanol puis est séché sous vide à 50°C.
Le produit du titre, d'une pureté de 99%, est obtenu sous forme d'une poudre blanche.
Rendement : 87,3 %
PF = 189,5°C
RMN ¹H (303 kHz, méthanol-d₄) δ (ppm) : 2,93 (complexe, 2H), 3,35 - 3,42 (complexe, 2H), 3,59 - 3,64 (complexe, 2H), 3,66 (large t, J = 5,13, 2H), 4,08 (large s, 2H), 6,24 (ddd, J = 6,89, 3,46, 1,62, 1H), 7,34 (complexe, 2H), 7,37 (complexe, 1H), 7,57 (t, J = 7,70, 1H), 7,62 (d, J = 7,72, 1 H), 7,72 (d, J = 7,91, 1 H), 7,73 (s, 1 H), 7,79 (dd, -, 1,72, 1 H), 7,81 (dd, 2H), 7,82 (d, J = 5,57, 1 H), 7,94 (d, J = 8,50, 1 H), 8,05 (large s, 1H), 8,42 (d, J = 5,82, 1 H), 9,20 (s,1H)

### Comparaison des propriétés physico-chimiques obtenues avec différents sels.

### Exemple 2 : Stabilité chimique.

On a étudié la stabilité chimique du composé de formule (1) sous forme de dichlorhydrate, de dihydrogénosulphate, de fumarate, de dibenzènesulfonate et de monobenzènesulfonate.
Chacun des cinq sels ci-dessus est exposé à la chaleur, à l'humidité et à la lumière dans les conditions ci-après :
- 14 jours à une température de 80°C,
- 14 jours à une température de 80°C et un taux d'humidité relative (HR) de 80%,
- une exposition à la lumière de 400 W.Hrs.m⁻².
Les résultats mesurés par chromatographie liquide à haute performance (HLPC) sont donnés dans le tableau I ci-après.

**Tableau I**

| **Conditions / sel** | **Dichlorhydrate** | **Dihydrogéno sulphate** | **Fumarate** | **DiBésylate** | **Bésylate** |
|---|---|---|---|---|---|
| Pureté initiale | 97,0 % | 93,3 % | 97,9 % | 97,5% | 98,2 % |
| 14 jours à 80°C | 95,6 % | 91,9 % | 96,7 % | 97,5% | 97,9 % |
| 14 jours à 80°C / 80 % HR | 92,9 % | 88,5 % | 32,4 % | 96,2% | 96,5 % |

Ainsi qu'il apparaît, le composé de formule (I) sous forme de bésylate montre une stabilité chimique à la chaleur parmi les plus élevées.
Le composé (I) sous forme de bésylate est également parmi les sels les moins dégradés lors des tests réalisés en température et en humidité.

### Exemple 3 : Teneur en eau.

La teneur en eau des cinq sels ci-dessus a été étudiée par analyse thermo-gravimétrique (ATG).
Les essais ATG sont réalisés sur un appareil TA8000 équipé d'un module TGA850. Les calibrations en température sont réalisées classiquement en utilisant la fusion de l'indium.
Les paramètres expérimentaux employés étaient :
Température de départ : 25°C
Rampe de chauffage : 10°C/min
Température finale : 250°C pour le dichlorhydrate, 300°C pour le fumarate, 200°C ou 250°C pour le dihydrogénosulfate, 250°C ou 300°C pour le dibésylate et le bésylate.
La purge du système est réalisée en utilisant de l'azote à un débit de 70 mL/min.
Les creusets utilisés étaient des creusets de 70µl Silice.
Les résultats sont donnés dans le tableau II ci-après.

**Tableau II**

| **Sel** | **Perte de masse (% pds/pds)** | **Nature du solvant** |
|---|---|---|
| Dichlorhydrate | 11,0 | Eau |
| Dihydrogénosulphate | 2,06 | Solvant + Eau |
| Fumarate | 0,45 | Solvant + Eau |
| Di-bésylate | 0,72 | Eau |
| Bésylate | 0,11 | Eau |

La courbe ATG du dichlorhydrate montre que le sel contient trois molécules d'eau par mole de sel anhydre et peut être considéré comme existant sous forme de trihydrate. La perte d'eau apparaît à relativement basse température, ce qui traduit un caractère faiblement lié de cet hydrate. Une telle forme peut s'avérer désavantageuse, la stoechiométrie pouvant varier en fonction des conditions ambiantes. Ce type de résultat traduit potentiellement une probabilité pour que le principe actif puisse exister sous différentes formes hydratées.
Le fumarate montre une petite perte de masse entre 25°C et 110°C en ATG, qui est attribuée à du solvant résiduel et à de l'eau faiblement liée. De manière similaire, le dihydrogénosulfate et le dibésylate présentent des pertes de masse en ATG dues à du solvant résiduel ou de l'eau faiblement liée, suivies à haute température par la décomposition du produit.
Le dihydrogénosulphate et le dibésylate montrent une acquisition significative d'eau pendant le cycle de sorption entre 30 et 80% d'humidité relative, ce que l'on cherche généralement à éviter pour une composition pharmaceutique. Les hystérésis observés lors de la désorption montrent un changement de la forme physique du produit étudié, ce qui est généralement non désiré pour une substance active.

Les essais DVS sont réalisés en utilisant un système DVS-1. De 20 à 32 mg de produit sont utilisés par analyse et la température est maintenue à 25°C plus ou moins 0,2°C. L'azote est utilisé comme gaz vecteur au débit de 100 mL/min dans la chambre d'échantillon. L'humidité relative évolue en suivant des cycles de 30% à 95% puis à 0%, pour terminer à 30% par incréments lorsque les conditions d'équilibre (dm/dt) de 0,0002 % sont atteintes sur une période de 5 min.

Les isothermes de sorption du fumarate montrent une hygroscopicité faible de ce sel. Les isothermes de sorption du dichlorhydrate montrent que 1,9% poids/poids d'eau est acquis pendant le cycle de sorption entre 30 et 80% d'humidité relative. Cette sorption significative reste toutefois acceptable, d'autant que l'on observe un hystérésis faible sur l'ensemble du domaine de travail entre 20% et 95% HR.
Le bésylate en revanche n'est pas hydraté et montre un excellent comportement lors des mesures d'isotherme de sorption d'eau. Ce sel apparaît comme étant le moins hygroscopique de tous les sels testés. Aucun hystérésis n'est enregistré lors de la mesure DVS en sorption et en désorption, ce qui le rend potentiellement intéressant pour un développement pharmaceutique.

II ressort de ces analyses que le sel bésylate du composé de formule (I) présente à la fois des propriétés d'hygroscopicité et de stabilité, que ce soit à la chaleur sèche ou humide, supérieures aux autres sels testés, et qui le rendent particulièrement adapté à la fabrication de médicaments.
Les propriétés physico-chimiques du composé de formule (I) sous forme bésylate permettent également sa conservation dans les conditions usuelles sans précautions trop contraignantes en rapport avec la présence de lumière, la température et l'humidité.

Le bésylate de 7-(2-(4-(3-trifluoromethyl-phenyl)-1,2,3,6-tetrahydro-pyrid-1-yl)ethyl) isoquinoleine présente des propriétés modulatrices du TNF-alpha. A ce titre, il peut être utilisé pour la préparation de médicaments, en particulier de médicaments destinés à traiter les maladies liées à des troubles immunitaires ou inflammatoires.

De telles maladies comprennent l'athérosclérose, les maladies auto-immunes, les maladies qui entraînent la démyélinisation des neurones (telles que la sclérose en plaques), l'asthme, l'arthrite rhumatoïde, les maladies fibrotiques, la fibrose idiopathique pulmonaire, la fibrose cystique, la glomérulonéphrite, la spondylite rhumatoïde, l'ostéoarthrite, la goutte, la résorbtion osseuse et cartilagineuse, l'ostéoporose, la maladie de Paget, le myélome multiple, l'uvéorétinite, les chocs septiques, la septicémie, les chocs endotoxiniques, la réaction du greffon contre l'hôte, le rejet des greffes, le syndrome de détresse respiratoire de l'adulte, la silicose, l'asbestose, la sarcoïdose pulmonaire, la maladie de Crohn, la colite ulcérative, la sclérose latérale amyotrophique, la maladie d'Alzheimer, la maladie de Parkinson, le lupus érythémateux disséminé, les chocs hémodynamiques, les pathologies ischémiques (infarctus myocardique, ischémie myocardique, vasospasme coronarien, angine de poitrine, insuffisance cardiaque, attaque cardiaque), les atteintes post ischémiques de reperfusion, la malaria, les infections mycobactériennes, la méningite, la lèpre, les infections virales (HIV, cytomegalovirus, virus de l'herpès), les infections opportunistes liées au sida, la tuberculose, le psoriasis, la dermatose atopique et de contact, le diabète, la cachexie, le cancer, les dommages liés aux radiations.

Ainsi, selon un autre de ses aspects, l'invention a pour objet des médicaments qui comprennent le bésylate de 7-(2-(4-(3-trifluoromethyl-phenyl)-1,2,3,6-tetrahydropyrid-1-yl)ethyl) isoquinoleine.
Ces médicaments trouvent leur emploi en thérapeutique, notamment dans le traitement et la prévention des indications cliniques liées à une dégradation du système immunitaire ou à une inflammation, ou dans lesquelles des complications inflammatoires ou immunitaires pourraient intervenir.

Un autre objet de l'invention est donc l'utilisation du bésylate de 7-(2-(4-(3-trifluoromethyl-phenyl)-1,2,3,6-tetrahydro-pyrid-1-yl)ethyl) isoquinoleine pour la préparation d'un médicament destiné à traiter les maladies liées à des troubles immunitaires ou inflammatoires.

Selon un autre de ses aspects, la présente invention concerne des compositions pharmaceutiques comprenant, en tant que principe actif, le bésylate de 7-(2-(4-(3-trifluoromethyl-phenyl)-1,2,3,6-tetrahydro-pyrid-1-yl)ethyl) isoquinoleine. Ces compositions pharmaceutiques contiennent une dose efficace de bésylate de 7-(2-(4-(3-trifluoromethyl-phenyl)-1,2,3,6-tetrahydro-pyrid-1-yl)ethyl) isoquinoleine, ainsi qu'au moins un excipient pharmaceutiquement acceptable. Lesdits excipients sont choisis selon la forme pharmaceutique et le mode d'administration souhaités, parmi les excipients habituels qui sont connus de l'Homme du métier.

Ces compositions pharmaceutiques peuvent être présentées sous toutes formes appropriées à l'administration orale, parentérale ou intraveineuse, telles que comprimés, dragées, gélules, capsules, suspensions ou solutions buvables ou injectables, etc., en association avec des excipients convenables. Toutes ces formes sont avantageusement dosées pour permettre une administration de 1 à 1000 mg par jour et par patient, en une ou plusieurs doses. II peut y avoir des cas particuliers où des dosages plus élevés ou plus faibles sont appropriés ; de tels dosages ne sortent pas du cadre de l'invention. Selon la pratique habituelle, le dosage approprié à chaque patient est déterminé par le médecin selon le mode d'administration, le poids et la réponse dudit patient.
A titre d'exemple, une forme unitaire d'administration sous forme de comprimé peut comprendre les composants suivants :
- Bésylate de 7-(2-(4-(3-trifluoromethyl-phenyl)-1,2
   3,6-tetrahydro-pyrid-1-yl)ethyl) isoquinoleine 50,0 mg
- Mannitol 223,75 mg
- Croscaramellose sodique 6,0 mg
- Amidon de maïs 15,0 mg
- Hydroxypropyl-méthylcellulose 2,25 mg
- Stéarate de magnésium 3,0 mg

La présente invention, selon un autre de ses aspects, concerne également une méthode de traitement des pathologies cliniques liées à une inflammation ou à une dégradation du système immunitaire, ou dans lesquelles des complications inflammatoires ou immunitaires pourraient intervenir, qui comprend l'administration à un patient, d'une dose efficace de bésylate de 7-(2-(4-(3-trifluoromethyl-phenyl)-1,2,3,6-tetrahydro-pyrid-1-yl)ethyl) isoquinoleine.

## Revendications

1. Sel bésylate de 7-(2-(4-(3-trifluoromethyl-phenyl)-1,2,3,6-tetrahydro-pyrid-1-yl)ethyl) isoquinoleine.

2. Procédé de préparation du bésylate de 7-(2-(4-(3-trifluoromethyl-phenyl)-1,2,3,6-tetrahydro-pyrid-1-yl)ethyl) isoquinoleine, **caractérisé en ce que** l'on fait réagir la 7-(2-(4-(3-trifluoromethyl-phenyl)-1,2,3,6-tetrahydro-pyrid-1-yl)ethyl) isoquinoleine en solution dans l'éthanol avec une solution d'acide benzène sulfonique dans l'éthanol.

3. Médicament, **caractérisé en ce qu'il** comprend le bésylate de 7-(2-(4-(3-trifluoromethyl-phenyl)-1,2,3,6-tetrahydro-pyrid-1-yl)ethyl) isoquinoleine.

4. Composition pharmaceutique, **caractérisée en ce qu'elle** comprend, à titre de principe actif, le bésylate de 7-(2-(4-(3-trifluoromethyl-phenyl)-1,2,3,6-tetrahydro-pyrid-1-yl)ethyl) isoquinoleine, ainsi qu'au moins un excipient pharmaceutiquement acceptable.

5. Utilisation du bésylate de 7-(2-(4-(3-trifluoromethyl-phenyl)-1,2,3,6-tetrahydropyrid-1-yl)ethyl) isoquinoleine pour la préparation d'un médicament destiné à traiter les maladies liées à des troubles immunitaires ou inflammatoires.

6. Utilisation selon la revendication 5, pour la préparation d'un médicament destiné à traiter l'arthrite rhumatoïde.

## Claims

1. 7-(2-(4-(3-(Trifluoromethyl)phenyl)-1,2,3,6-tetrahydropyrid-1-yl)ethyl)isoquinoline besylate salt.

2. Process for the preparation of 7-(2-(4-(3-(trifluoromethyl)phenyl)-1,2,3,6-tetrahydropyrid-1-yl)ethyl)isoquinoline besylate, **characterized in that** 7-(2-(4-(3-(trifluoromethyl)phenyl)-1,2,3,6-tetrahydropyrid-1-yl)ethyl)isoquinoline, in solution in ethanol, is reacted with a solution of benzenesulphonic acid in ethanol.

3. Medicament, **characterized in that** it comprises 7-(2-(4-(3-(trifluoromethyl)phenyl)-1,2,3,6-tetrahydropyrid-1-yl)ethyl)isoquinoline besylate.

4. Pharmaceutical composition, **characterized in that** it comprises, as active principle, 7-(2-(4-(3-(trifluoromethyl)phenyl)-1,2,3,6-tetrahydropyrid-1-yl)ethyl)isoquinoline besylate and at least one pharmaceutically acceptable excipient.

5. Use of 7-(2-(4-(3-(trifluoromethyl)phenyl)-1,2,3,6-tetrahydropyrid-1-yl)ethyl)isoquinoline besylate in the preparation of a medicament intended to treat diseases related to immune or inflammatory disorders.

6. Use according to Claim 5 in the preparation of a medicament intended to treat rheumatoid arthritis.

## Patentansprüche

1. Besylatsalz von 7-(2-(4-(3-Trifluormethylphenyl)-1,2,3,6-tetrahydropyrid-1-yl)ethyl)isochinolein.

2. Verfahren zur Herstellung von 7-(2-(4-(3-Trifluormethylphenyl)-1,2,3,6-tetrahydropyrid-1-yl)ethyl)isochinolein-Besylat, **dadurch gekennzeichnet, dass** man 7-(2-(4-(3-Trifluormethylphenyl)-1,2,3,6-tetrahydropyrid-1-yl)ethyl)isochinolein in Lösung in Ethanol mit einer Lösung von Benzolsulfonsäure in Ethanol umsetzt.

3. Arzneimittel, **dadurch gekennzeichnet, dass** es 7-(2-(4-(3-Trifluormethylphenyl)-1,2,3,6-tetrahydropyrid-1-yl)ethyl)isochinolein-Besylat umfasst.

4. Pharmazeutische Zusammensetzung, **dadurch gekennzeichnet, dass** sie als Wirkstoff 7-(2-(4-(3-Trifluormethylphenyl)-1,2,3,6-tetrahydropyrid-1-yl)ethyl)isochinolein-Besylat sowie mindestens einen pharmazeutisch annehmbaren Excipienten umfasst.

5. Verwendung von 7-(2-(4-(3-Trifluor-methylphenyl)-1,2,3,6-tetrahydropyrid-1-yl)ethyl)isochinolein-Besylat zur Herstellung eines Arzneimittels zur Behandlung von Krankheiten in Verbindung mit Immun- oder entzündlichen Störungen.

6. Verwendung nach Anspruch 5 zur Herstellung eines Arzneimittels zur Behandlung von rheumatoider Arthritis.
